# EUROPEAN PATENT APPLICATION

(11) **EP 0 941 691 A1**
(43) Date of publication of application: **15.09.1999**
(21) Application number: 99301787.0
(22) Date of filing: 09.03.1999
(51) Int. Cl.: A61B 1/247, A61B 1/05

(54) **Compact video imaging assembly**

(30) Priority: 11.03.1998 US 77523 P
(71) Applicant: WELCH ALLYN, INC., Skaneateles Falls New York 13153 (US)
(72) Inventor: Wood, Robert, J., Syracuse, NY 13215 (US)
(74) Representative: Evens, Paul Jonathan

(57) **Abstract**

A compact diagnostic video imaging system includes in a cylindrical housing (168) a focusing lens (178), a solid state sensor (174) and support electronics on an IC board (180) for converting the optical signal into a corresponding electrical signal. Preferably, the solid state sensor (174) is a compact low power CMOS video camera. A plurality of white-light producing LEDs (190) preferably disposed adjacent to the solid state sensor (174) and disposed planarly with imaging optics (178) are used to direct light onto the target area. Preferably, the totality of the imaging system including the signal processing electronics (180) and illuminating optics (190) are situated in a space envelope (168) which is not larger than the periphery of the solid state sensor (174), allowing the imaging system to be located within the distal end of an electronic viewing instrument.

## Description

### FIELD OF THE INVENTION

The invention relates to the field of videoized instruments, and in particular to a compact electronic imaging and illumination assembly for use in a medical or other suitable diagnostic instrument.

### BACKGROUND OF THE INVENTION

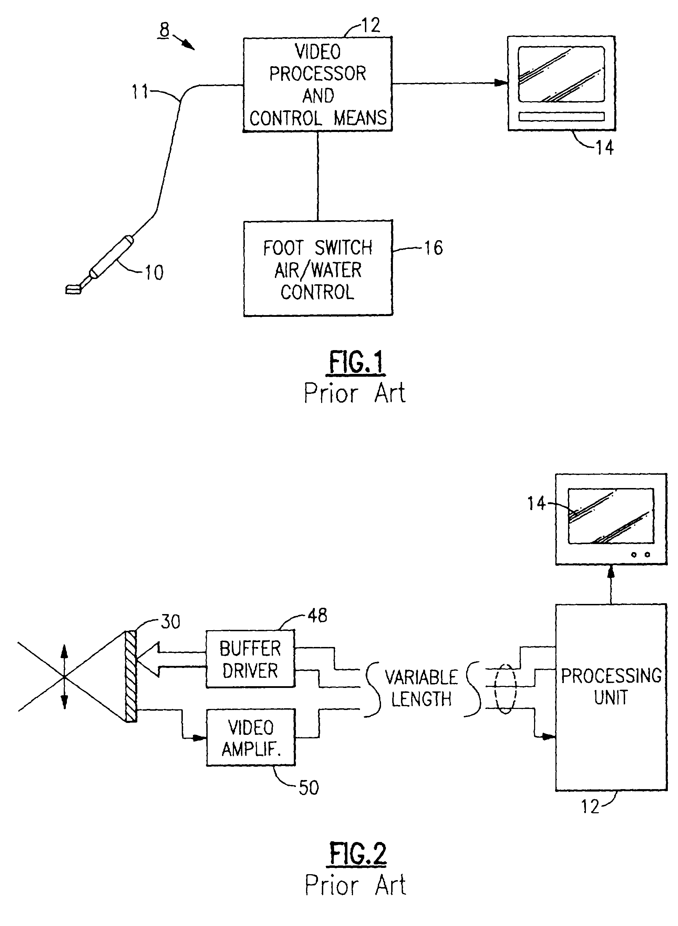

The prior art includes numerous examples of electronic video imaging systems used in connection with diagnostic or other forms of instruments, A typical example is described in U.S. Patent No. 5,016,098, issued to Cooper et al, and shown in relevant part according to Figs. 14. Referring specifically to Fig. 1, the instrument system 8 is used for the examination of a patient's teeth and includes a dental camera 10. A video processor and control means 12 provides power, light and, if desired, fluid and other appropriate signals via a cable 11 to the dental camera 10. The video processor and control means 12 also provides a video signal to a video monitor 14 in order that users may view a location within a patient's mouth using the dental camera 10. If desired, a foot switch 16 is used to provide a signal to the video processor and control means 12 indicating when air/fluids are to be sent to the dental camera in a manner known in the field.

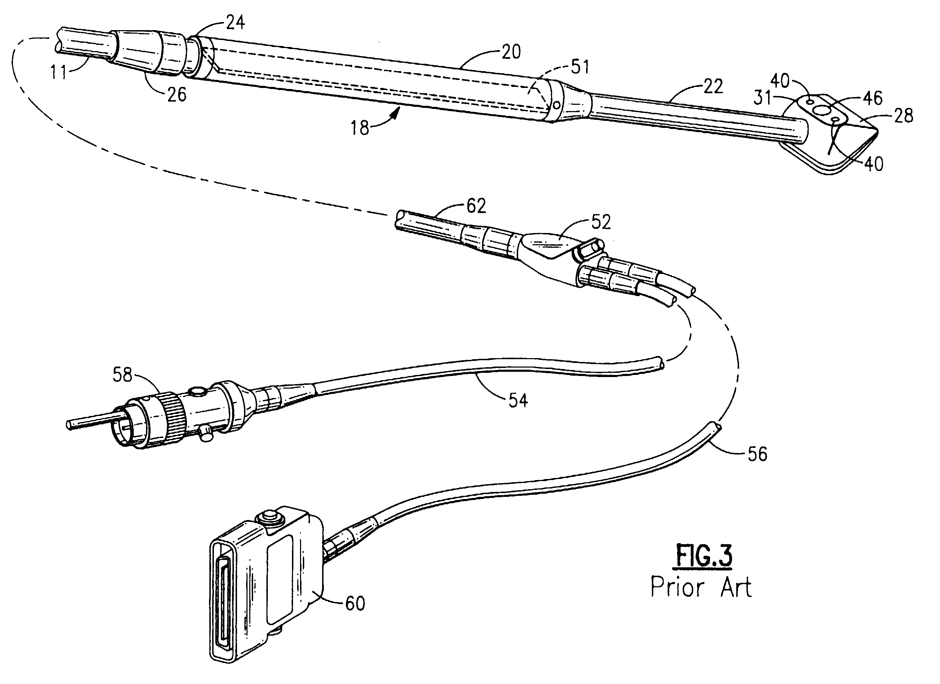

Briefly, and referring to Figs. 1 and 3, the above dental camera 10 includes a body 18 having an elongated handle 20 suitable for being held by an operator and a forward extension or neck 22. At one end 24 of the handle 20 is located a connector 26 allowing connection by the cable 11 to the video processor and control means 12.

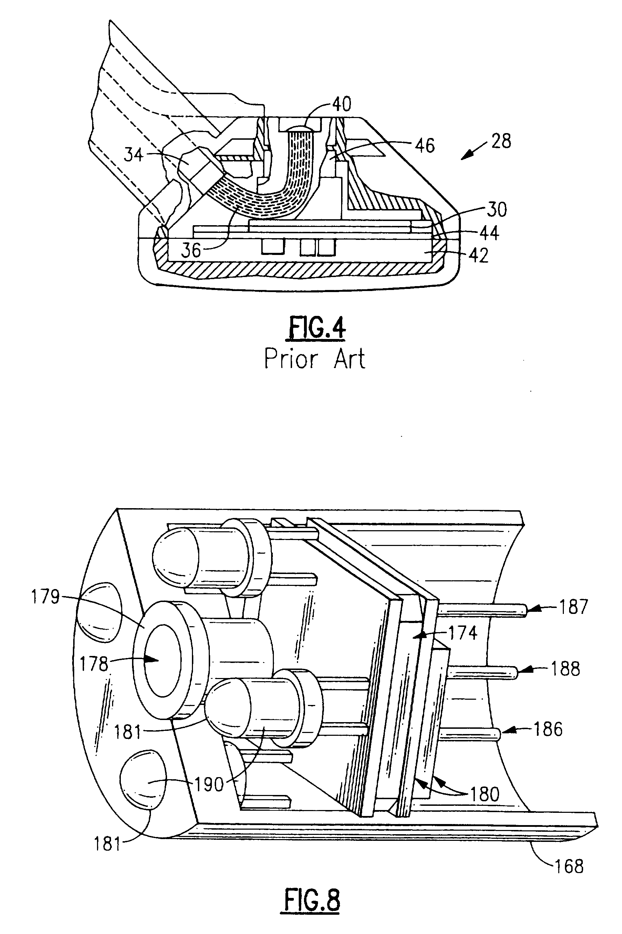

Referring to Figs. 1, 3 and 4, a camera head 28 is mounted on the distal end of the neck portion 22, the camera head including a face 31 for receiving an image within a patient's mouth to be displayed on the video monitor 14. An image sensor 30 is disposed within a cavity 42 formed within the camera head 28, while fiber optic light guides 34 are used to receive light from a light source (not shown) which may comprise, for example, a halogen lamp or a xenon arc lamp disposed at the proximal end of the light guides 34, and conduct the light through a curvature 36 of the light guides 34 to an illuminating lens assembly 40.

The image sensor 30, which is a solid-state electronic sensor, such as a charge coupled device (CCD) is mounted on a hybrid assembly substrate 44 located within a cavity 42 of the camera head 28. An image lens 46 is mounted to the image sensor 30 in a fixed relationship, while the light guides 34 are disposed on either side of the image sensor 30, as shown.

Referring to Fig. 2, a block diagram of the electronics portion of the dental camera system 8 is illustrated which includes the image sensor (CCD) 30, an image device drive buffer 48 that serves to control the image sensor 30, signal amplifier circuitry 50, and the video processor 12 for controlling the driver buffer 48 and processing the signal information into a monitor ready video signal. Typically, the drive buffer 48 and the amplifier 50 are placed on a circuit board or maintained as part of an integrated or hybrid circuit. In the above description, each are provided on the hybrid substrate 44. Additional circuitry is provided on a flexible circuit board 51 disposed in the handle 20, the board having regulators and drivers for powering the image sensor 30.

Referring to Fig. 3, the cable 11 containing a number of electrical connectors for transmitting power and the amplified electrical signal and the bundle of light guides 34 is connected to a connector 52 wherein the power and video portions are extended into a separate cable 56. The light guides 34 are extended into another separate cable 54, each of the cables extending from the connector 52. A port 58 at the end of the cable 54 allows interconnection with a light box (not shown) to allow coupling of the light guides with a source of illumination, while cable 56 includes a connector 60 at the opposite end thereof which engages the video processor 12, which typically also acts as a power supply. Alternately, the power supply can be provided in the light box, as is known.

In use, the camera head 28 is placed into the mouth of the patient (not shown) and an illuminated optical image is focused onto the light receiving surface of the image sensor 30. The image sensor 30 then creates an electrical signal which is amplified by the circuitry 50 contained in the hybrid substrate 44 and the handle 20, the signal being transmitted through the cables 11, 56 to the video processing electronics contained in the remotely disposed processor 12. Illumination is provided from a remote light source (not shown) and requires the bundle of light guides 34 to channel the light through the cables 54, 11 to the distal camera head 28.

As is apparent from the preceding Figs., a number of discrete components are required to provide signal processing, power, and illumination to the diagnostic instrument. In addition, the light guides 34 are integrally provided as part of the above system 8, the guides being epoxied within the instrument interior. Because of the relative fragility of the light guides and the construction of the above assembly, any breakage or malfunction thereof usually requires replacement of the entire instrument, including the imaging system.

Other similar prior art videoized diagnostic instrument systems are described in U.S. Patent No. 5,051,823 to Cooper et al., relating to a similar intraoral dental camera system, and U.S. Patent Nos. 5,050,584 to Matsuura, 5,634,790 to Pathmanabhan et al., and 4,918,521 to Yabe et al, relating to videoized endoscopic instrument systems face problems that are similar to those encountered by the '098 reference above.

Diagnostic instruments have been developed which attempt to deal with the above problems. Such a system, described in commonly assigned U.S. Patent No. 5,527,262, issued to Monroe et al., is depicted in Figs. 5 and 6. This instrument system 70, in general, also includes a hand-hold able videoized diagnostic instrument 72. A video signal is transmitted from the instrument 72 through a cable 74 attached at one end 76 to the proximal end of the instrument and at a remaining opposite end to a connector 78, which is coupled through a strain relief to a power supply box 80 adapted to be plugged into a common wall outlet 79 to provide power to the instrument. A video cable 82 couples a video monitor 84 to the cable 74 through a wiring harness (not shown) inside the power supply box 80.

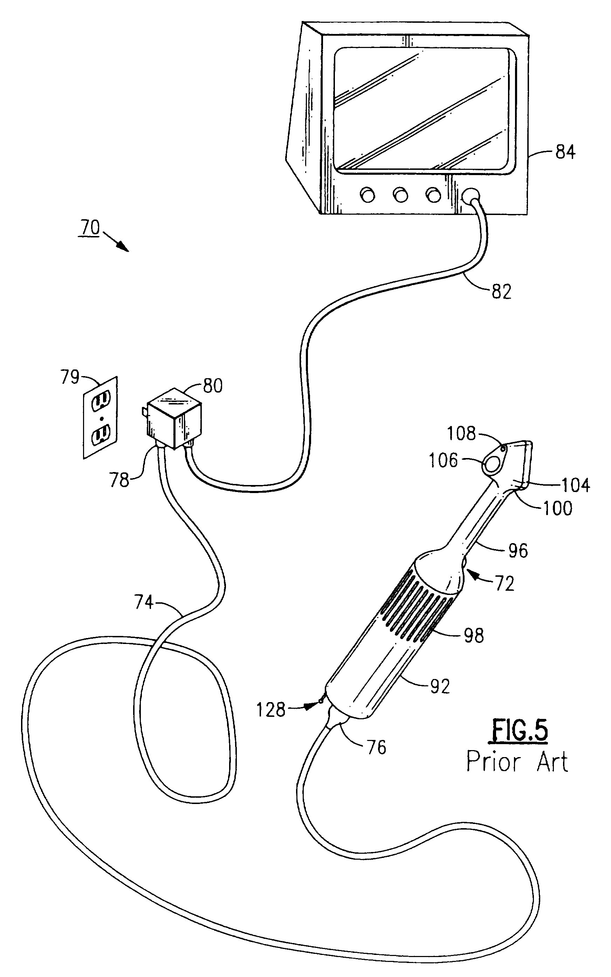

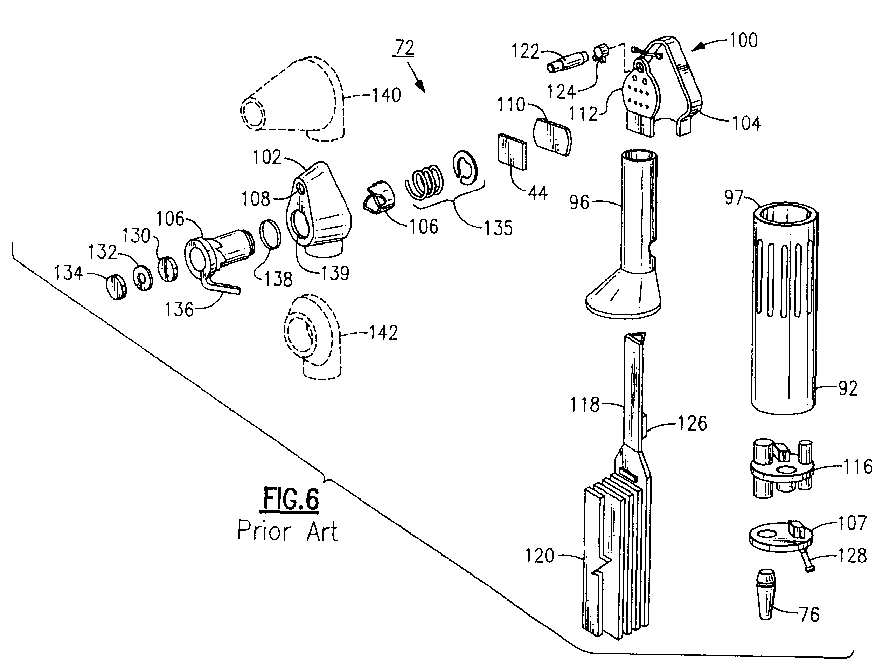

Details of the instrument 72 are shown in Fig. 5 and the exploded Fig. 6. The instrument 72 includes a hand-hold able casing 88 with a cylindrical body portion 92, a neck portion 96 that extends from the upper end 97 of the body portion, and a head portion 100. The head portion 100 is oval in shape and is formed from a front cover 102 and a back cover 104. An adjustable lens cell 106 is situated on the head portion 100 in the front cover 102 and a lamp window 108 is provided above the lens cell 106.

A miniature CCD imager chip 110 is mounted on a circuit board 112 disposed within the head portion 100 of the casing 88 with an infrared filter 114 being positioned over the light receiving surface thereof. The active light receiving surface (not shown) of the imager chip 110 is aligned with the lens cell 106. An elongated and flexible circuit board 118, similar to that described in the '098 reference, is connected to the circuit board 112, and carries flexible circuitry for powering the imager, the board extending longitudinally through the elongated neck portion 96 of the casing 88, while a plurality of separately disposed circuit boards 120 are located within the hollow interior of the body portion 92. Finally, a disc-shaped printed circuit board 116 is disposed adjacent an end cap 107 which carries an on/off switch 128 for powering the instrument 72, as well as the cable end 76. In this instrument, the boards 112, 116, 118 and 120 contain the entirety of the video processing circuitry, as opposed to using a separate video processor. That is to say, the video processmg circuitry is not provided externally from the instrument, as shown in the above described '098 system.

A miniature lamp 122 is fitted into a lamp holder 124 mounted at one end of the circuit board 112. Activation of the lamp 122 is controlled independently through use of an on/off lamp switch 126, the lamp being positioned to direct illumination forward through the window 108 in the front cover 102 and into the field of view of the distal lens cell 106. The lens cell includes a number of optical components including an objective lens 130, an aperture plate 132, and a plano lens 134 which are disposed in a distal end thereof, the lens cell having a lever 136 to allow focusing and sealed in a fluid-tight manner using a ring seal 138 against a biasing means 135.

The above system additionally allows interchangeability, wherein the front cover 102 is used for dental examination, or alternately front covers 140, 142 can be substituted for otoscopic and ophthalmoscopic applications, respectively.

Though the above assembly minimizes the number of component parts of the overall video diagnostic instrument assembly as compared, for example, with the above prior art instrument systems, there are associated problems. First, the miniature lamp and the CCD imager chip each have relatively high power consumptions. In addition, the lamp develops significant heat build-up in use, requiring separate means to be developed for dissipating the heat, making conversion of the assembly using the alternate front covers difficult. The use of a single lamp also fails to provide uniform illumination depending on the target of interest. The instrument is also quite heavy owing to the number of circuit boards required to power the imager and illumination assembly.

There is a desire in the field to provide an imaging system which allows the entirety of the video camera, including the signal processing circuitry, to be modularly disposed in a diagnostic or other suitable instrument.

There is a desire to improve the state of the art of video imaging assemblies.

There is a further desire to provide a video imaging assembly which is sufficiently compact to allow the entire assembly, including imaging optics, illuminating optics and all video processing circuitry, to be conveniently positioned within the insertion portion of a diagnostic or other suitable viewing instrument.

There is yet a further desire to provide a compact video imaging assembly suitable for placement in literally any form or design of a video instrument, in which the only needed input is power for the imaging assembly, and in which video output is directly transmitted from the compact video assembly in the form of a signal which is suitable for viewing by a video monitor, without any additional processing being required.

There is still a further desire to provide a compact imaging system having an efficient source of illumination which can be disposed, preferably within the envelope of the imaging assembly, without recourse to separate exterior illumination sources, fiber-optic bundles or other apparatus.

There is yet a further desire to provide an imaging assembly which is releasably connected to a diagnostic instrument wherein replacement or repair of the imaging assembly is convenient and can be performed without requiring replacement of the instrument.

Therefore and according to a preferred aspect of the invention, there is provided a compact video imaging assembly including an electronic sensor having a light receiving surface for receiving an optical signal of a target of interest, means for focusing the optical signal onto the light receiving surface, and video processing electronics for completely converting the optical signal into a monitor-ready video signal. Preferably, the electronic sensor is a CMOS imaging element having processing electronics disposed in an integrated circuit integral with the imaging element. An electronic support disposed in immediate proximity to the electronic sensor includes components for powering the imager chip and for directing the processed video signal therefrom.

The imaging assembly further includes illumination means disposed adjacent to the imaging element which are preferably disposed planarly with the focusing optics. Preferably, the entire imaging assembly represented by the signal processing electronics, electronic sensor and illuminating optics are sized to fit within a space envelope which is no larger than the periphery of the IC board of the electronic sensor, allowing convenient locating of the imaging assembly within the instrument head of a video instrument.

According to another preferred aspect of the present invention, there is provided a compact imaging assembly used in conjunction with a video diagnostic instrument, wherein the diagnostic instrument includes a housing having a distal end and a proximal end, said housing having a hollow interior, the imaging system including:
an electronic sensor having an imaging substrate capable of receiving an optical image and for converting the image into an electrical signal;
video processing means for fully processing and converting the optical signal into a monitor-ready video signal; and
means for illuminating a target of interest, wherein the illuminating means, video processing means and electronic sensor are each located in the instrument head portion of the diagnostic instrument.

According to yet another preferred aspect of the present invention, there is provided a dental intraoral instrument comprising:
an instrument housing including an elongated body portion having a distal end and a proximal end, and a camera head attached to said distal end;
video camera means including an electronic sensor having an imaging substrate; and
signal processing means for fully processing an optical signal received by said video camera means, wherein the entirety of said video camera means and said signal processing means are disposed in said camera head such that a processed video signal can be directly output therefrom.

An advantage provided by the described video imaging system is that the entirety of the assembly; that is, the imaging element and all related circuitry required to convert an incoming optical signal into an a suitable for viewing video signal are contained within the camera head.

Another advantage of the present invention is that the entire illumination system, including the illuminaton source, can also be positioned within the camera head in an extremely compact and efficient manner. Therefore, an entire imaging assembly previously requiring several components can be situated in a space envelope which is orders of magnitude smaller than any previously contemplated.

A further advantage realized are the relatively few number of inputs and outputs required. The only external interconnection required for the described assembly is for delivering electrical power into the system and for transmitting the fully processed video signal from the instrument to a peripheral device.

Yet another advantage of the present invention is that by providing an efficient illumination source, such as an array of white-light emitting LEDs, within the distal end of the instrument, provides a compact, low power and efficient light source, deleting the need for optical fiber bundles or other extemal illumination source, and greatly simplifying the manufacture and maintenance of an instrument having the described system.

Another advantage of the present invention is that the entire imaging system, including the processing circuitry can be detachably mounted and removed from the instrument of use, allowing simple and inexpensive replacement and repair, as needed without having to tear down or scrap the entire instrument.

These and other objects, features, and advantages will now be described with reference to the following Detailed Description of the Invention, which should be read in conjunction with the accompanying drawings.

### BRIEF DESCRIPTION OF THE DRAWINGS

Fig. 1 is a diagrammatic view of a diagnostic instrument system in accordance with the prior art;
Fig. 2 is a block diagram of the electronics portion of the prior art diagnostic instrument of Fig. 1;
Fig. 3 is a partial perspective view of the prior art instrument system of Figs. 1 and 2;
Fig. 4 is a sectional view of the camera head of the diagnostic instrument of Figs. 1-3;
Fig. 5 is a perspective view of a second prior art diagnostic instrument system;
Fig. 6 is an exploded assembly view of the prior art diagnostic instrument of Fig 5;
Fig. 7 (a) is a perspective view, respectively, partially in section, of a diagnostic instrument system having a video imaging assembly made in accordance with a first embodiment of the present invention;
Fig. 7(b) is an enlarged portion of Fig. 7(a), illustrating the video imaging assembly in accordance with the present invention;
Fig. 8 is a further enlarged and isometric cutaway view of the video imaging assembly used in the diagnostic instrument system of Figs. 7(a) and 7(b);
Fig. 9 is a partial electrical schematic of the imaging assembly of Fig. 6;
Fig. 10 is an electrical schematic of an alternative imaging assembly in accordance with the present invention;
Fig. 11 is a side view, partially in section of an intraoral dental camera having the video imaging assembly of Figs. 7-9;
Fig. 12 is a front perspective view, partially cutaway, and having the video imaging assembly of Figs. 7-9; and
Figs. 13(a) and 13(b) are front perspective and enlarged cutaway views of a borescope having the video imaging assembly of Figs. 7-9.

### DETAILED DESCRIPTION

The following description relates to preferred embodiments which include certain diagnostic applications, including an intraoral dental camera, an endoscope, and a borescope. It will be readily apparent, however, from the discussion that follows that the described imaging assembly can be utilized in literally any videoized instrument, including but not limited to medical diagnostic instruments such as ophthalmoscopes, otoscopes, and other endoscopes, as well as other viewing instruments having medical and non-medical uses, such as optical bar-code readers and scanners, among others.

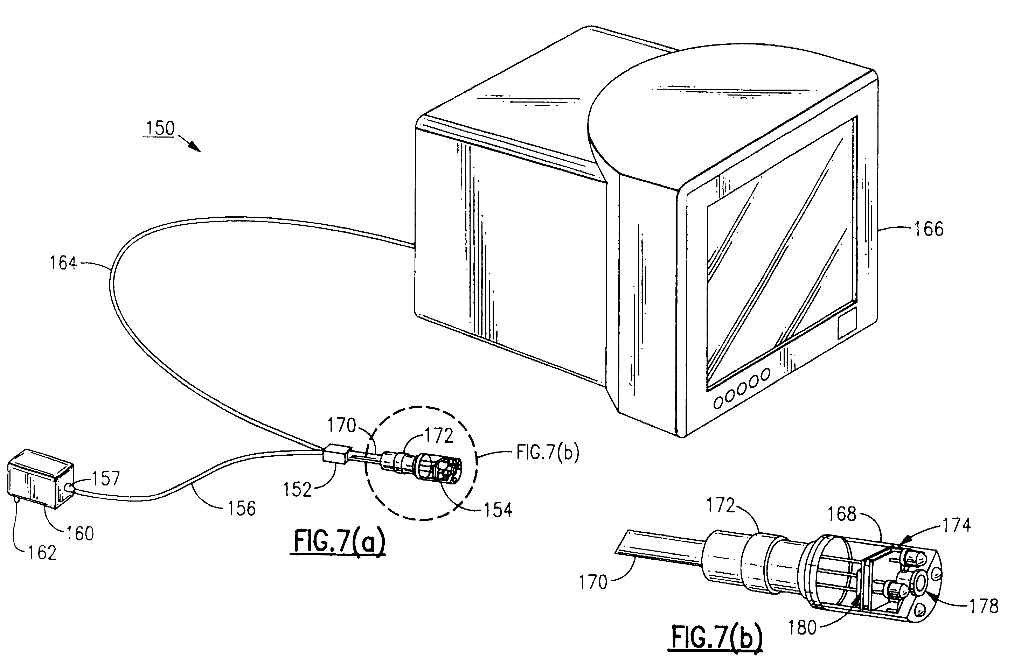

Referring now to Fig. 7(a), a generalized view of a viewing instrument system 150 utilizing a compact video imaging assembly 153 according to the present invention is depicted. The instrument system 150 includes a viewing instrument 154 attached by means of a sheathed electrical cable 156 to a power supply unit 160 via a connector 157. The power supply unit 160 includes plugs 162 (partially shown) adapted for connection to a wall outlet (not shown). Similarly, a video cable 164 also extends from the instrument 154 to a video monitor 166. Each of the cables 156, 164 are spliced into a single instrument cable 170 through a suitable connector 152, the instrument cable being attached by known means to a suitable connector 172 at the proximal end 158 of the instrument 152. Though a video monitor is shown, it should be apparent that any video peripheral device, such as a video printer, video tape player, computer monitor or any device capable of receiving a video signal can be substituted or used in the described system.

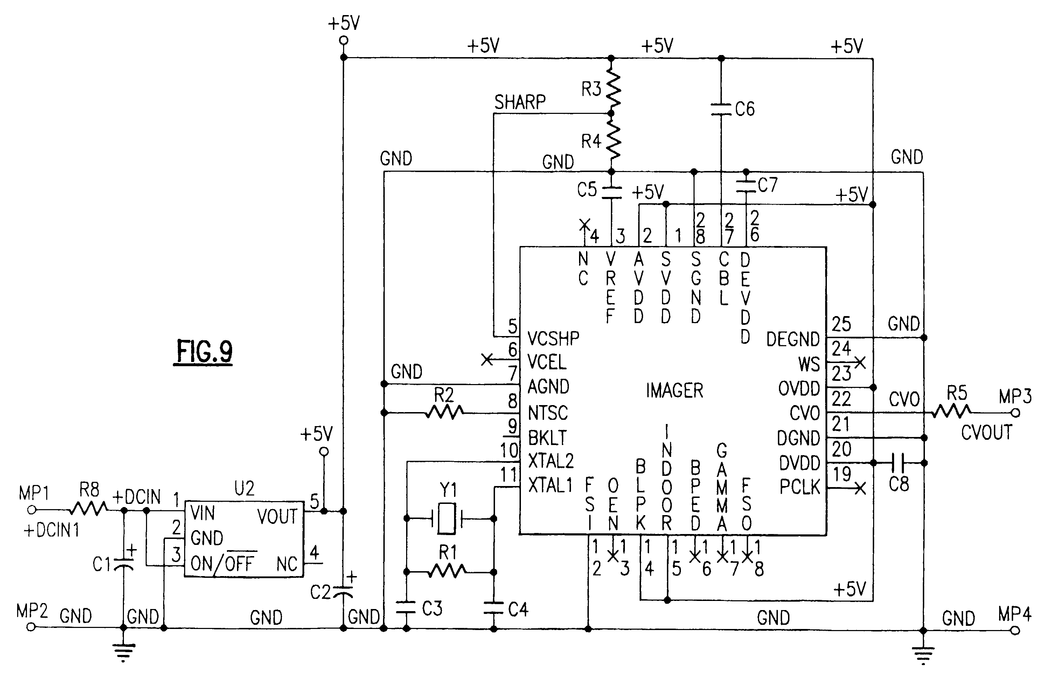

Figs. 7(b) and 8 illustrate enlarged views of the diagnostic instrument 154 which, according to this embodiment, is defined by a compact cylindrical housing 168 and an interior 172 sized for retaining the imaging assembly 153 of the present invention. The imaging assembly 153 includes three major components: an electronic imager 174, support circuitry in the form of a printed circuit board 180 disposed at the rear side of the imager 174, and a focusing lens cell 178. In the present embodiment, the imager chip 174 is a Model OV-5016 single IC black and white analog video camera manufactured by Omnivision Technologies, Inc. of Sunnyvale, California. In brief, the imager chip 174 is a low power imager having a photodiode-based sensor array, an analog signal processing block, and a digital processing block. The sensor array is an active light receiving surface disposed as part of an imaging substrate and very compact processing circuitry which are contained entirely within an integrated circuit to allow an optical signal supplied by a focusing lens cell 174 to be fully converted into a monitor ready NTSC or other suitably formatted video signal.

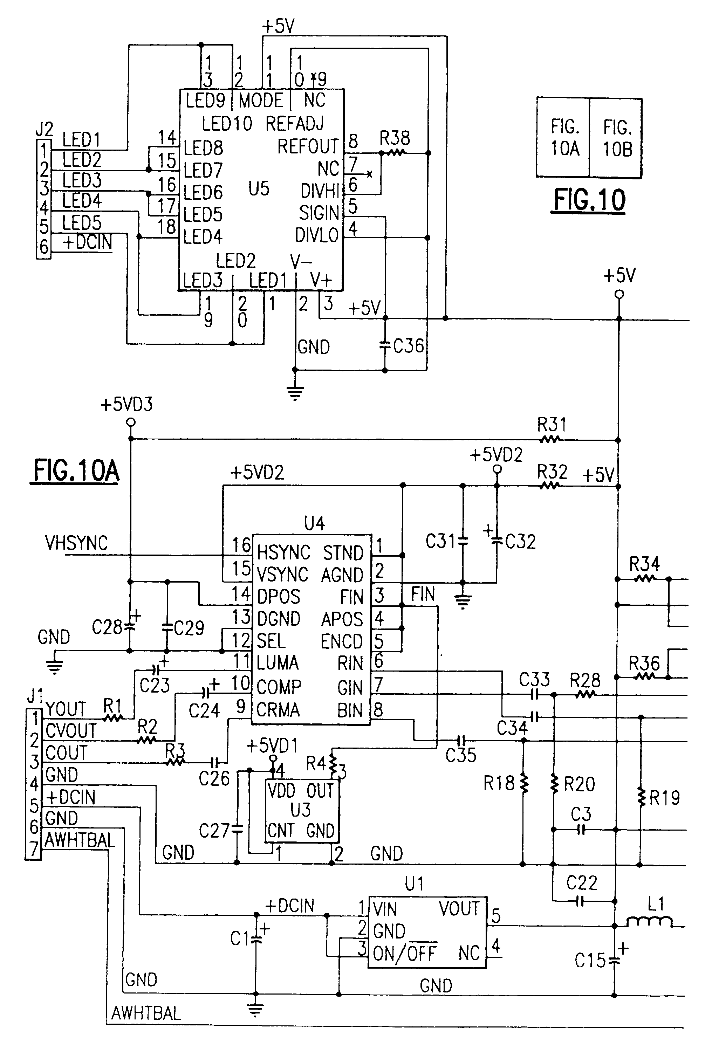

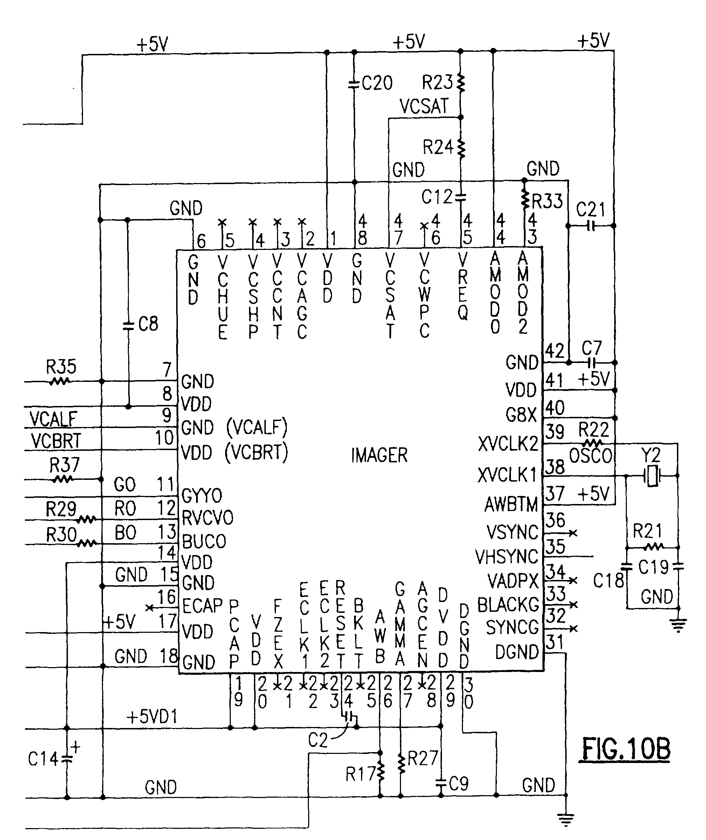

Other suitable imager chips can be alternately substituted, such as a color version of the above analog camera, Model No. OV-6003, also manufactured by Omnivision Technologies, Inc. An electrical schematic of the above camera is attached as Fig. 10. It should be realized that other imaging elements or sensors can also be used, though the above types each utilize CMOS architecture and have low power requirements typically in the range of approximately +5.0 +15.0 VDC.

The processing circuitry 180 according to this embodiment includes a single printed circuit board which is soldered or otherwise fixedly attached to the rear side of the above imager chip 174 and containing the necessary elements including a suitable crystal oscillator for dnving the imager, as well as regulators and drivers, as shown in Fig. 9. The board 180 further includes a plug connector, shown schematically in Fig. 9, for receiving power from an extemal or internal source, in this instance power supply unit 160, and for transmitting a fully processed video signal to the monitor 166 or other peripheral device Significantly, the circuit board 180 according to this embodiment is sized within the periphery of the imager IC chip 174 and therefore allows a convenient fit within the instrument housing 168.

Three pins 186, 187, and 188 extending from the printed circuit board 180 are attached to electrical connectors in the instrument cable 172, the pins respectively providing ground, power in from the power supply unit and video out from the instrument 154. No other inputs or outputs are required for the imaging assembly 153. An electrical schematic of the above imaging system is provided as shown in the accompanying Fig. 9

The lens cell 178 consists of an objective lens system including at least one objective lens and an aperture plate (not shown) supported in a lens barrel 179 which is disposed in a center opening 181 of the cylindrical housing 168. The lens cell 178 is aligned so as to allow an optical image of a target being viewed to be focused upon the active surface of the imager 174. Alternately, the lens cell 178 can be mounted to the imager or provided as a detachable adapter (not shown) which can be mounted to the distal face of the housing 168.

Still referring to Figs. 7(b) and 8, the above instrument 154 also includes a plurality of solid state white-light producing LEDs (light emitting diodes) 190, each LED being provided in a spaced opening 192 on the distal face 194 of the instrument housing 168. The LEDs used according to this embodiment are Model *No.* NSPW 310AS, and manufactured by NICHL Corp. of Japan, utilizing a phosphor technology to emit white light having a higher color temperature than other known sources of white light. Either a bulb-type or a surface mount of the LEDs 190 is acceptable. According to this embodiment, the LEDs 190 are disposed in a circumferential arrangement relative to the active image window of the imager chip 174 and are substantially coplanar with the optical axis of the lens cell 178. According to the present embodiment, four (4) LEDs 190 are used, though this number can be easily varied, the LEDs each being electrically interconnected to the described circuit through lead wires (not shown) extending to the printed circuit board 180. An electrical arrangement for the LEDs 190 is shown in the electrical schematic of Fig. 10.

It should be apparent that other mounting schemes are possible. For example, a circular plate of plastic or of an optical grade material (not shown) can be added to the exterior of the distal face of the housing so as to uniformly distribute the light to the target.

In operation, and still referring to Figs. 7-9, the only necessary inputs to the described imaging system are that of electrical power and grounding. The presently described CMOS imager 174 requires low power in comparison to other solid state imaging elements, such as CCD imagers, as in known diagnostic instrument systems. In the present embodiment, +9.0 volts are sufficient to power the LEDS 190 and imager 174. The video output from the assembly 153 can include NTSC, as well as composite or other video formats.

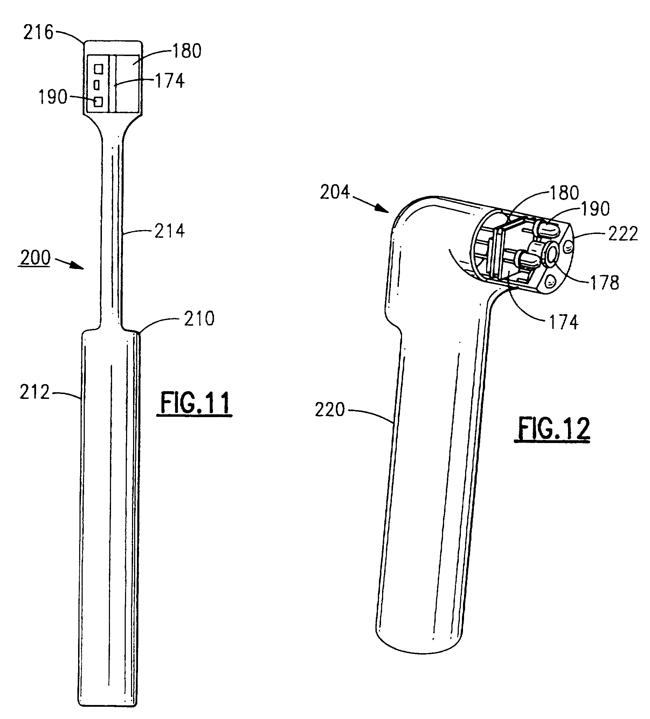

Figs. 11-13 illustrate different and alternate uses of the described imaging assembly 153 relative to an intraoral dental camera 200, a hand-held medical diagnostic instrument 204, and a borescope 208, respectively.

Referring to Fig. 11, the intraoral dental camera 200 includes a body portion 210 including a handle 212, an elongate neck portion 214 extending from one end of the handle and a camera head 216 attached to the neck portion. Each of the preceding features are similar, for example, to those described in the previously referenced '098 patent. The neck portion 214 and camera head 216 are each sized for insertion into the mouth of a patient (not shown). Preferably, the instrument is made from a material which allows sterilization after each patient use. The instrument, including the head is fluid-sealed to allow the device, including the contained imaging assembly to be autoclaved.

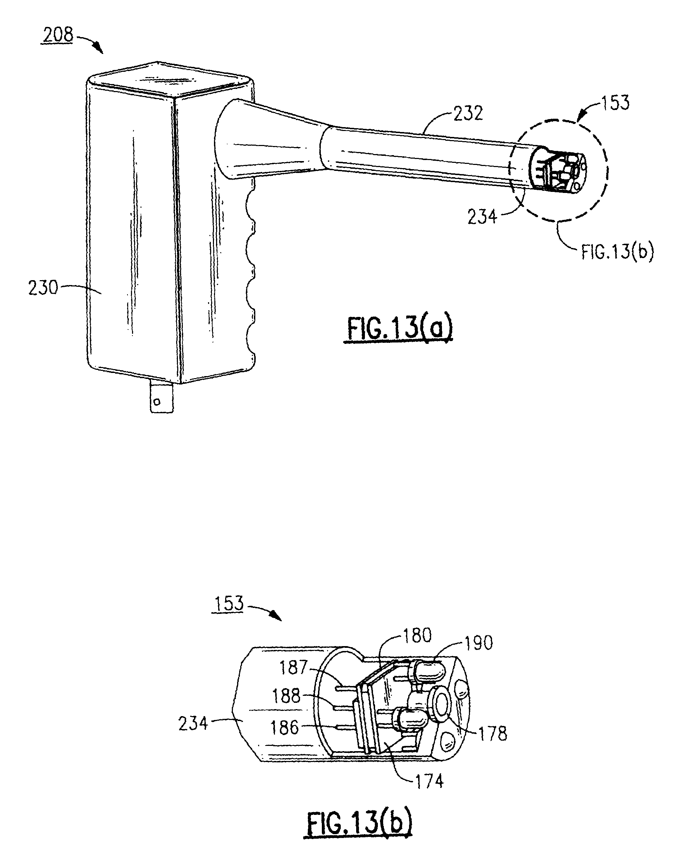

Referring to Fig. 12, the hand-held instrument 204 representative of a hand-held medical diagnostic instrument such as an otoscope, includes a unitary body portion 220 having an extending upper portion 222 having an interior which is sized for retaining the above-described imaging assembly 153.

Referring to Figs. 13(a) and 13(b), the borescope 208 includes a hand-grippable handle section 230 and an extending tubular portion 232 having a distally arranged instrument head 234 used for inspecting areas having limited access, such as pressure vessels, etc. The imaging assembly 153 of the present invention can be similarly retained within the instrument head 234.

In each instance, the workings of the instruments are substantially identical wherein the entirety of the imaging assembly 153, including the processing and powering circuitry 180, is included as an integral compact unit in the head of each instrument.

Though the present invention has been described in terms of a single embodiment, it will be readily apparent that other modifications and variations are possible within the spirit and scope of the invention. For example, instruments having contained power sources, such as batteries, can also be modified to utilize the described imaging system. In addition, the video signal can be alternately output using radio frequency or other wireless or cableless techniques. In a preferred embodiment, a miniature RF or IR image transmitter can also be disposed within the handle making the instrument entirely cableless. Another potential feature of the present invention is that the entirety of the imaging system 153 can be releasably attached to any diagnostic instrument, such as those shown in Figs. 7-13(b) and can be easily removed, providing significant flexibility in their use.

## Claims

1. A compact video imaging assembly for a viewing instrument, said instrument including a housing having an interior and a distal end, said assembly comprising:
an electronic sensor disposed in the distal end of said housing, said sensor including a light receiving surface for receiving an optical signal from a target of interest;
focusing means for focusing the optical signal onto said light receiving surface;
first signal processing means for converting said optical signal into an electrical signal;
illuminating means for illuminating the target of interest; and
second signal processing means for converting said electrical signal into a video signal, characterized in that said first and second signal processing means are sized to fit within the periphery defined of said electronic sensor, and in which said illuminating means at least one light source disposed adjacent a distal side of said light receiving surface.

2. A compact video imaging assembly according to Claim 1, further characterized in that said illuminating means includes a plurality of LEDs disposed adjacent to said focusing means in the distal end of said instrument.

3. A compact video imaging assembly according to Claim 1, further characterized in that said at least one LED of said plurality is a white light producing LED.

4. A compact video imaging assembly according to Claim 2, further characterized in that said LEDs are circumferentially disposed about said focusing means.

5. An imaging assembly according to Claim 3, further characterized in that said LEDs are circumferentially disposed about said focusing means.

6. A compact video imaging assembly according to Claim 3, further characterized in that said focusing means includes at least one focusing lens disposed in front of said light receiving surface of said electronic sensor, said at least one focusing lens and said light receiving surface defining a viewing axis, said instrument including an opening for allowing light to enter said instrument through said viewing axis.

7. A compact video imaging assembly according to Claim 6, further characterized in that said at least one focusing lens is retained within a lens barrel, said barrel being supported within said instrument.

8. A compact video imaging assembly according to Claim 7, further characterized in that said plurality of LEDs are disposed circumferentially between said focusing lens and the exterior of said imaging substrate of said electronic sensor.

9. A compact video imaging assembly according to Claim 1, further characterized in that said electronic sensor is a CCD.

10. A compact video imaging assembly according to Claim 1, further characterized in that said electronic sensor is a compact CMOS analog camera.

11. A compact video imaging assembly according to Claim 1, further characterized in that said instrument is a compact intraoral dental camera.

12. A compact video imaging assembly according to Claim 1, further characterized in that said instrument is a borescope.

13. A compact video imaging assembly according to Claim 1, further characterized in that said instrument is an endoscope.

14. A compact video imaging assembly according to Claim 1, further characterized in that said Instrument is a medical diagnostic instrument.

15. A compact video imaging assembly, comprising:
an electronic sensor including an IC chip having a light receiving surface for receiving an optical signal from a target of interest;
focusing means for focusing the optical signal onto said light receiving surface of said electronic sensor;
first signal processing means for converting said optical signal into an electrical signal;
illuminating means for illuminating the target of interest; and
second signal processing means for converting said electrical signal into a video signal, characterized in that said first and second signal processing means are disposed on a circuit board sized within the periphery defined of said electronic sensor, and in which said illuminating means at least one light source disposed adjacent the front side of said light receiving surface.

16. A compact video imaging assembly as recited in Claim 15, further characterized in that said electronic sensor is a CCD.

17. A compact video imaging assembly according to Claim 15, further characterized in that said electronic sensor is a CMOS imaging chip.

18. An intraoral dental camera for examining a patient's teeth, said camera comprising:
an instrument housing including an elongated body portion having a distal end and a proximal end, and a camera head attached to said distal end;
video camera means including an electronic sensor having an imaging substrate; and
signal processing means for fully processing an optical signal received by said video camera means, characterized in that the entirety of said video camera means and said signal processing means are disposed in said camera head such that a processed video signal can be directly output therefrom.

19. An intraoral dental camera according to Claim 18, further characterized in that said camera is autoclavable.

20. A compact video imaging assembly, comprising:
an electronic sensor including an IC chip having a light receiving surface for receiving an optical signal from a target of interest;
focusing means for focusing the optical signal onto said light receiving surface of said electronic sensor;
video signal processing means for converting said optical signal into a monitor-ready video signal; and
illuminating means for illuminating a target of interest, characterized in that said signal processing means are disposed on a circuit board sized within the periphery defined of said electronic sensor, and in which said illuminating means at least one light source disposed adjacent the front side of said light receiving surface.
